(19) Europäisches Patentamt
European Patent Office
Office européen des brevets

(11) EP 2 198 847 A2

(12) EUROPEAN PATENT APPLICATION

(43) Date of publication:
**23.06.2010 Bulletin 2010/25**

(51) Int Cl.:
***A61K 8/35** (2006.01)* ***A61K 8/97** (2006.01)*
***A61Q 19/04** (2006.01)*

(21) Application number: **09252829.8**

(22) Date of filing: **18.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **19.12.2008 US 339550**
**30.11.2009 US 627164**

(71) Applicant: **Johnson & Johnson Consumer Companies, Inc.**
**Skillman, NJ 08558 (US)**

(72) Inventors:
- **Clemente, Rudy**
**Hoboken, NJ 07030 (US)**
- **Kiser, Melba L.**
**Noblesville, IN 46060 (US)**

(74) Representative: **Kirsch, Susan Edith et al**
**Carpmaels & Ransford**
**43-45 Bloomsbury Square**
**London WC1A 2RA (GB)**

# (54) Compositions comprising self-tanning agents and plant extracts

(57) The present invention relates to a composition for darkening the skin. The composition includes a self-tanning agent, and a plant extract selected from the group consisting of extract of pre-germinated soy seeds, walnut seed extract, mugwort extract, and combinations thereof. In another aspect, a method of darkening the skin includes topically applying the above composition to the skin.



Printed by Jouve, 75001 PARIS (FR)

## Description

This application is a continuation-in-part of US application 12/339,550 filed on December 19, 2008, the complete disclosure of which is hereby incorporated herein by reference for all purposes.

## FIELD OF THE INVENTION

A composition and method for darkening the skin is provided. The composition includes a self-tanning agent, and a skin-darkening effective amount an extract selected from the group consisting of extract of pre-germinated soy seeds, walnut tree extract, mugwort extract, and combinations thereof.

## BACKGROUND OF THE INVENTION

Many individuals desire the darkening of skin color. Most people obtain darker skin through exposure to UV light (e.g., sun tanning or UV lamps). UV exposure, however, results in accelerated skin aging and increased incidence of skin cancer. The ability to generate a tanned or darkened appearance without incurring photodamage, thus, is important. Accordingly, alternative methods for "sunless tanning" have evolved.

Products containing dihydroxy acetone (DHA) are well known as sunless tanners. These products, however, produce color that often develops either too slowly and/or is either or intense enough or has an appearance that is somewhat dissimilar from a natural "sunlight-derived" tan.

Applicants have unexpectedly discovered that extract of pre-germinated soy seeds, walnut tree extract, and mugwort extract provide skin darkening benefits when used topically in conjunction with a self-tanning agent.

## SUMMARY OF THE INVENTION

The invention provides a composition comprising: (a) a self-tanning agent having the formula:

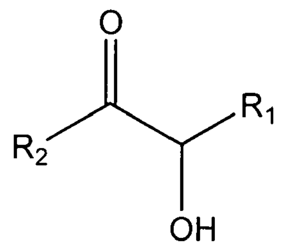

wherein $R_1$ is H, $CH_2OH$, $CHOHCH_2OH$, CH(OH)CH(=O), $CH(NH_2)CH(=O)$, $CH(OCH_3)CH(=O)$, or CH(NH-phenyl)CH (=O); and $R_2$ is H or $CH_2OH$; and (b) an extract selected from the group consisting of extract of pre-germinated soy seeds, walnut tree extract, mugwort extract, and combinations thereof. In one particular embodiment the extract includes an extract of pre-germinated soy seeds.

According to another aspect, the invention provides a method of darkening the skin, said method comprising the topical administering to the skin a composition comprising (a) a self-tanning agent having the formula:

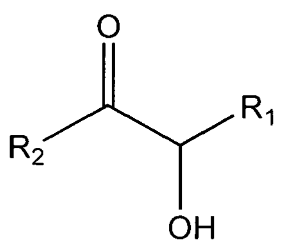

wherein $R_1$ is H, $CH_2OH$, $CHOHCH_2OH$, CH(OH)CH(=O), $CH(NH_2)CH(=O)$, $CH(OCH_3)CH(=O)$, or CH(NH-phenyl)CH (=O); and $R_2$ is H or $CH_2OH$; and (b) an extract selected from the group consisting of extract of pre-germinated soy seeds, walnut tree extract, mugwort extract, and combinations thereof.

Other features and advantages of the present invention will be apparent from the detailed description of the invention and from the claims.

DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

Compositions of the present invention are generally suitable for darkening the skin. As used herein, "darkening" is darkening the appearance of human skin or hair, including, but not limited to, darkening human skin to either achieve a "sun tan" effect or to cover light areas of the skin (e.g., as a result of a scar or a disease or a therapy) or darkening natural hair color or restoring discolored hair due to aging (e.g., gray or white hair) or external aggressions (e.g., excess exposure to sun or chlorine). While throughout the specification reference is made to darkening the skin, it is believed that other keratinous bodily materials such as hair may also be darkened.

As used herein, a "product" is a product in finished packaged form. In one embodiment, the package is a container such as a plastic, metal or glass tube or jar containing the composition. The product may further contain additional packaging such as a plastic or cardboard box for storing such container. In one embodiment, the product comprises a composition of the invention and contains instructions directing the user to apply the composition to the skin or hair to darken the skin (e.g., to tan the skin), even skin tone (e.g., to darken light areas of the skin or to treat or prevent mottled hyperpigmentation), or darken the hair (e.g., to darken light brown, blonde, gray or white hairs). Such instructions may be printed on the container, label insert, or on any additional packaging.

As used herein, "topically applying" means directly laying on or spreading on outer skin, the scalp, or hair, e.g., by use of the hands or an applicator such as a wipe, roller, or spray.

As used herein, "cosmetically acceptable" means that the ingredients the term describes are suitable for use in contact with tissues (e.g., the skin or hair) without undue toxicity, incompatibility, instability, irritation, allergic response, or the like.

As used herein, a "skin darkening effective amount" means an amount sufficient to induce a darkening of human skin or hair. This amount will vary with the area being treated, the age and skin or hair type of the end user, the duration and nature of the treatment, the specific composition employed, the carrier utilized, and like factors.

Self-Tanning Agent

The compositions of the invention comprise a self-tanning agent, that is, a chemical agent capable of producing or inducing the artificial tanning process of the skin by forming brown pigments in the skin, e.g., through the Maillard reaction reported in Bobin, et al., J. Soc. Cosmet. Chem., 35:265-72 (1984). In particular, the self-tanning agent is an α-hydroxy ketone or aldehyde of the formula:

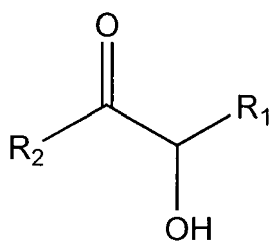

wherein $R_1$ is H, $CH_2OH$, $CHOHCH_2OH$, $CH(OH)CH(=O)$, $CH(NH_2)CH(=O)$, $CH(OCH_3)CH(=O)$, or CH(NH-Phenyl)CH (=O); and $R_2$ is H or $CH_2OH$. Examples of such a compound include 1,3-dihydroxyacetone (i.e., dihydroxyacetone, DHA) and 1,3,4-trihydroxy-2-butanone (i.e., erythulose). In a preferred embodiment, the self-tanning agent is 1,3-dihydroxyacetone.

The amount of self-tanning agent in the compositions of the invention varies from about 0.1 to about 10, preferably from about 1 to about 5, more preferably from about 2 to about 5 weight percent based on the total weight of

the composition.

Plant Extract

According to the invention, the composition also comprises a skin-darkening effective amount of a plant extract. The plant extract (phylum Plantae) is selected from the group consisting of extract pre-germinated soy seeds, walnut tree extract, mugwort extract and combinations thereof. Applicants have found that by utilizing the extracts above one is able to greatly enhance the effects of the self-tanning agent.

What is meant by an "extract from pre-germinated soy seeds" is a blend of compounds isolated from the ungerminated seed of the soy (soja) plant, e.g. from *Glycine soya).* The pre-germinated soy seed extract may be prepared in a gentle manner so as to omit one or more of certain steps (e.g., non-aqueous solvent extraction, alkali processing, and/or high temperature heating) commonly used to produce conventional total soy protein hydrolyzates.

In another embodiment, the process for extracting the soy protein is a so-called "single cell suspension" process that includes seperation of individual cells via an enzymatic process to degrade cellulose in the outer cell wall, but leaves inner cell walls intact.

One particularly suitable extract from pre-germinated soy seeds is commercially available from Gattefosse of Saint-Priest, France and sold under the name "Phylderm Vegetal C," and is described as an amino-peptidic complex produced from soya bean embryonic tissues prepared with enzymatic and molecular selection technics.

The amount of the extract from pre-germinated soy seeds present in the skin darkening composition of this invention is from about 0.001% to about 20%, and preferably from about 0.01% to about 10%, and even more preferably from about 1.5% to about 3% by weight of the composition.

By "walnut seed extract" it is meant an extract of a seed from any of the species of plants from the family Juglandaceae. Examples include: *Juglans regia, Juglans sigillata, Juglans australis, Juglans brasiliensis, Juglans californica, Juglans hindsii, Juglans hirsuta, Juglans jamaicensis, Juglans major, Juglans microcarp,* among others.

One particularly suitable walnut seed extract is a mixture of water and Juglans regia (walnut) seed having a 27% to 33% dry matter content, commercially available from Gattefosse of Saint-Priest, France and sold under the name "Gatuline Age Defense."

The amount of the walnut seed extract present in the skin darkening composition of this invention is from about 0.001 % to about 20%, and preferably from about 0.01% to about 10%, and even more preferably from about 1.5% to about 3% by weight, by weight of the composition.

By "mugwort extract" it is meant an extract of a plant from the genus *Artemesia,* such as *Artemesia* vulgaris and also referred to as common wormwood, felon herb, chrysanthemum weed, wild wormwood, or St. John's plant (not St. John's *wort*). The extract may be of any of various parts of the plant including seeds, leaves, stems, or other parts of the plant. The mugwort extract may be combined with extracts of other plants, such as, for example, a green algae extract. One particularly suitable mugwort extract is a mixture of mugwort extract and green algae (family, *Ulvaceae*) extract and, commercially available as "Triple A Complex," from Barnet Products Corporation of Englewood Cliffs, New Jersey.

The amount of the mugwort extract present in the skin darkening composition of this invention is from about 0.001% to about 20%, and preferably from about 0.01% to about 10%, and even more preferably from about 1.5% to about 3% by weight, by weight of the composition.

In one embodiment of the invention, the composition includes a self tanning agent such as DHA, a first plant extract, and a second plant extract. The first plant extract is selected from the group consisting of extract of mugwort, walnut tree extract, and combinations thereof. The first plant extract is particularly suited to providing enhanced darkening of skin in 3 hours or less following topical application. The second plant extract is extract of pre-germinated soy seeds. The plant second extract is particularly suited to providing enhanced darkening of skin in 24 hours after topical application. Thus, the resulting composition has darkening that, when compared to DHA alone, both develops more quickly and maintains a stronger intensity over an extended period of time.

Other Tanning Enhancing Ingredients

In one embodiment, compositions of the present invention include other tanning enhancing compounds, i.e., compounds other than plant extracts that are useful for enhancing the development of color/tanning. Examples of other tanning enhancing compounds include sugar amines. The sugar amine can be synthetic or natural in origin and can be a pure compound or mixtures of compounds (e.g., extracts from natural sources or mixtures of synthetic materials). Preferably, the sugar amine comprises glucosamine, N-acetyl glucosamine, mannosamine, N-acetyl mannosamine, galactosamine, N-acetyl galactosamine, isomers thereof (e.g., stereoisomers), salts thereof (e.g., HCl salt), or mixtures thereof. In a preferred embodiment, the sugar amine includes glucosamine. The cosmetic composition according to the invention may comprise from 0.01% to 15%, preferably from 0.1 % to 10%, and more preferably from 0.5% to 5% of at

least one sugar amine by weight of the composition.

Other Darkening Agents

In one embodiment, the topical composition further contains an additional darkening agent such as, but not limited to, lawsone, erythulose, melanin, peptides, synthetic melanin derivatives, vanillin polymers, pigments, extracts such as but not limited to Coleus Forskoli extract, extracts from natural sources containing pigments (e.g., brown pigments from plants from the Hedychium genus or Bearberry genus or yellow, orange and red pigments from plants containing carotenoids or canthaxanthins); or synthetic chemicals such as compounds containing copper (e.g., copper salts such as CuCl2) or synthetic carotenoids or canthaxantins. What is meant by an "extract" is a mixture of compounds isolated from a natural source (e.g., a plant).

Examples of synthetic melanin derivatives are disclosed in U.S. Patent Nos. 5,618,519, 5,384,116, and 5,227,459. Examples of soluble melanin derivatives are disclosed in US Patent Nos. 5,744,125, 5,225,435, 5,218,079, and 5,216,116. Examples of commercially available soluble melanin derivatives include Melasyn-100™ from Sanmar laboratories, Inc. (Elmsford, NY) and MelanZe™ from Zylepsis (Ashford, Kent, United Kingdom).

These additional darkening agents will typically be present in the composition in an amount from about 0.001 % to about 10% by weight.

In another embodiment, the composition may include a peptide. Examples of suitable peptides are described e.g. in US Patent Nos. 7,081,442; 7,214,655; 6,797,697; and 7,025,951.

Topical Compositions

The compositions of the present invention are applied topically to human skin or hair. In one embodiment, the composition contains a skin darkening effective amount of the self-tanning agent described above, an extract selected from the group consisting of extract of pre-germinated soy seeds, walnut tree extract, mugwort extract, and combinations thereof; and a cosmetically acceptable topical carrier. In one embodiment, the cosmetically acceptable topical carrier is from about 50% to about 99.99%, by weight, of the composition (e.g., from about 80% to about 99%, by weight, of the composition). In a preferred embodiment of the invention, the cosmetically acceptable topical carrier consists essentially of or includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol (200-600), polypropylene glycol (425-2025), glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Although it is preferred that the composition of the present invention includes water, the composition may alternatively be anhydrous or an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1 % to about 2% of a thickening agent(s). Examples of thickening agents include, but are not limited to, those set forth in the ICI Handbook pp. 2979-84.

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1 % and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

<u>Additional Cosmetically Active Agents</u>

In one embodiment, the composition further contains another cosmetically active agent. As used herein, a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, titanium dioxide, octyl salicylate, homosalate, avobenzone, carotenoids, free radical scavengers, spin traps, amines (e.g., DMAE and neutrol), retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof. The cosmetically active agent will typically be present in the composition of the invention in an amount of from about 0.001% to about 20% by weight of the composition, e.g., about 0.005% to about 10% such as about 0.01% to about 5%.

Examples of vitamins include, but are not limited to, vitamin A, vitamin Bs such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

Other Materials

Various other materials may also be present in the composition, as known in the art. These include humectants, pH adjusters, chelating agents (e.g., EDTA), minerals, and preservatives (e.g., parabens). Examples of such agents are listed in pp. 2922-23, 2926-28, and 2892 of the ICI Handbook. In addition, the compositions useful herein can contain conventional cosmetic adjuvants, such as opacifiers (e.g., titanium dioxide), and fragrances.

Dyes may also be suitable to use in compositions of the present invention. Examples of dyes suitable for the compositions of the invention include caramel, carmine, fluorescein derivatives, methoxsalen, trioxsalen, carbon black, azo dyes, anthraquinone dyes, blue azulenes, guajazulene, chamuzulene, erythrosin, bengal rose, phloxin, cyanosin, daphinin, eosin G, cosin 10B, Acid Red 51, Red Dye 4, Red Dye 40, Blue Dye 1, and Yellow Dye 5, or mixtures thereof. Other dyes are listed on page 1628-30 of the International Cosmetic Ingredient Dictionary and Handbook, eds. Wenninger and McEwen (The Cosmetic, Toiletry, and Fragrance Assoc., Washington, D.C., 7th Edition, 1997) (hereinafter "ICT Handbook"), the contents of which are incorporated herein by reference.

When used, the amount of dye in the composition may vary from about 0.0001 to about 0.1, preferably about 0.0025 to about 0.025, weight percent based on the total weight of the composition.

The composition and formulations and products containing such compositions of the present invention may be prepared using methodology that is well known by an artisan of ordinary skill.

The following non-limiting examples further illustrate the invention:

Example A: Mugwort extract and walnut seed extract provide superior 3 hour skin tanning (darkening)

A base ("Comp. 1") was prepared with a concentration of 1.5% by weight dihydroxyacetone (DHA), propylene glycol, water and the remainder ethanol. A series of similar compositions were prepared with the same concentrations of DHA, propylene glycol, water, and ethanol, however, for each of the additional compositions 2% of a particular extract was also included.

The following extracts were evaluated: Gatuline Age Defense (mixture of water and Juglans regia (walnut) seed, commercially available from Gattefosse of Saint-Priest, France), Triple A Complex (mixture of mugwort extract and *Ulvaceae* green algae extract, commercially available from Barnet Products Corporation of Englewood Cliffs, New Jersey), Vegerysyl LP (a conventionally processed hydrolyzed soy protein), and soy milk NA22077 (a conventionally processed total soy protein hydrolyzate, commercially available from Crodarom of Les Plaines, Chanac, France).

Each of these compositions was tested according to the IN-VIVO SKIN DARKENING TEST for their ability to induce tanning/skin darkening in human subjects.

IN-VIVO SKIN DARKENING TEST

Two human subjects having Fitzpatrick Skin Types I-III, age ranging from 21-50 were identified. For each subject, a location for each of the compositions to be tested was determined and initial chromometer X readings were taken using a Minolta Chromometer, available from Konica Minolta Sensing of Ramsey, NJ. These readings were averaged to arrive at a baseline reading for each subject. Each of the compositions described above were applied to one distinct area of the forearm for each subject at a coverage of 18 mg over 9 $cm^2$ of skin area and was rubbed in. The compositions were allowed to dry and after three time periods: 3 hours, 6 hours, and 24 hours, additional chromometer readings were taken. The change in intensity (dL) was calculated by subtracting the average reading after treatment to average reading of baseline.

The dL readings were recorded. In addition, in order to allow for better comparisons between tests done at different time periods, the percent increase in darkening for each was normalized to that of the control composition (Comp. 1) which had the DHA and base, but no extract. This normalized change in intensity was calculated as:

$$\%\, dL_{norm} = 100^{*}\ (dL - dL_{Comp.\ 1}) / dL_{Comp.\ 1}$$

The results of the test are shown in Table 1.

Table 1

| Example | Description | dL (3 hr) | % $dL_{norm}$ (3 hr) | dL (6 hr) | % $dL_{norm}$ (6 hr) | dL (24 hr) | % $dL_{norm}$ (24 hr) |
|---|---|---|---|---|---|---|---|
| Comp. 1 | No extract | 0.118 | 0.0 | -1.474 | 0.0 | 3.190 | 0.0 |
| Ex. 1 | Gatuline Age Defense | -0.605 | 411 | -1.506 | 2.2 | -3.625 | 13.6 |
| Ex. 2 | Triple A Complex | -0.320 | 170 | -1.689 | 14.6 | -3.050 | -4.4 |
| Comp. 2 | Vegerysyl | -0.0673 | 43.2 | -0.485 | -67.1 | -3.075 | -3.6 |
| Comp. 3 | Soy Milk NA22077 | 0.5342 | 54.9 | -0.918 | -37.7 | -3.617 | 13.4 |

As can be clearly seen in the % $dL_{norm}$(3 hr), the walnut seed extract (Gatuline Age Defense) and mugwort extract (Triple A Complex) provided quick acting darkening effects. When independently combined with DHA, these extracts act within 3 hours to provide dramatic boost in darkening over the DHA alone. The % $dL_{norm}$(3 hr) of 411 for the composition including Gatuline Age Defense indicates that it provided 4.11 times the amount of darkness as compared to the same composition without any extract (Comp. 1). Similarly, Triple A Complex provided 1.70 times the darkening effect of Comp. 1. Furthermore, the darkening effect measured by % $dL_{norm}$(3 hr) was much higher (about 4 to 10 times better) for Ex. 1 and Ex. 2 as compared to Comp. 2 and Comp. 3 (containing other extracts - Vegerysyl conventional soy and conventional soy milk, respectively).

Example B: Extract from pre-germinated soy seeds provides superior 24 hour skin tanning (darkening)

A second evaluation was performed, similar to the evaluation Example A above. The base ("Comp. 2.2") was prepared with a concentration of 1.5% dihydroxyacetone (DHA), deionized water, propylene glycol, and the remainder ethanol. A series of similar compositions were prepared with the same concentrations of DHA and oil blend, however, for each of the additional compositions 2% of a particular natural extract was also included. The following extracts were evaluated: Phylderm Vegetal C (a pre-germinated soy seed extract, available from Gattefosse of Saint-Priest, France), Sea Silk (a water hydrolyzed green algae extract of Enteromorpha compressa and Himantalia elongate, available from Biotech Marine of Poitrieux, France), Phycolanine ( a water laminaria ochroleuca, brown seaweed extract, also available from Biotech Marine), and Aosaine (a hydrolyzed Ulva lactuca, green seaweed extract, also available from Biotech Marine).

Table 2

| Example | Description | dL (3 hr) | % $dL_{norm}$ (3 hr) | dL (6 hr) | % $dL_{norm}$ (6 hr) | dL (24 hr) | % $dL_{norm}$ (24 hr) |
|---|---|---|---|---|---|---|---|
| Comp. 2-2 | No extract | -1.569 | 0.0 | -3.056 | 0.0 | -2.284 | 0.0 |
| Ex. 3 | Phylderm Vegetal C | -1.422 | -9.4 | -2.594 | -15.1 | -3.116 | 36.5 |
| Comp. 2-3 | Sea Silk | -1.751 | 11.7 | -2.903 | -5.0 | -2.583 | 13.1 |
| Comp. 2-4 | Phycolanine | -1.610 | 2.7 | -2.070 | -32.3 | -2.542 | 11.3 |
| Comp. 2-5 | Aosaine | -0.350 | -77.7 | -1.140 | -62.7 | -1.519 | -33.5 |

As can be clearly seen in the % $dL_{norm}$(24 hr) the pre-germinated soy seed extract (Phylderm Vegetal C) provided intense and lasting darkening effects. When independently combined with DHA, this extract acted in 24 hours to provide dramatic boost in darkening over the DHA alone. The % $dL_{norm}$(24 hr) of 36.5 for the composition including Phylderm Vegetal C (Ex. 3) indicates that it boosted the darkening ability by 36.5%, as compared to the same composition without any extract (Comp. 2-2). At 24 hours, this boost was nearly 3 times the boost from the best of the remaining other extracts (Gatuline and conventional soy milk in Example A and Sea Silk and Phycolanine in Example B). It should also be noted that some of the comparative extracts, Aosine and Vegerysyl, actually retarded the development of darkening at 24 hours (% improvement over DHA was negative).

It is understood that while the invention has been described in conjunction with the detailed description thereof, that the foregoing description is intended to illustrate and not limit the scope the invention, which is defined by the scope of the appended claims. Other aspects, advantages, and modifications are within the claims.

## Claims

1. A composition comprising:

   a self-tanning agent having the formula:

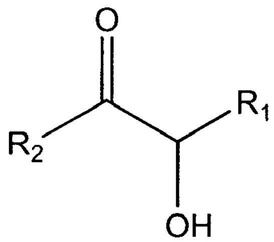

   wherein $R_1$ is H, $CH_2OH$, $CHOHCH_2OH$, $CH(OH)CH(=O)$, $CH(NH_2)CH(=O)$, $CH(OCH_3)CH(=O)$, or CH(NH-phenyl)CH(=O); and $R_2$ is H or $CH_2OH$; and
   an extract selected from the group consisting of extract of pre-germinated soy seeds, walnut tree extract, mugwort extract, and combinations thereof.

2. The composition of claim 1, comprising extract of pre-germinated soy seeds.

3. The composition of claim 1, comprising walnut tree extract.

4. The composition of claim 1, comprising mugwort extract.

5. The composition of any preceding claim, wherein the self-tanning agent is dihydroxy acetone.

6. The composition of any preceding claim, wherein the self-tanning agent is present in the composition in an amount from about 1 % by weight to 5% by weight of the composition.

7. The composition of any preceding claim, wherein the extract is present in the composition in an amount from about 0.01 % by weight to about 10% by weight of the composition.

8. The composition of any preceding claim, wherein the extract is present in the composition in an amount from about 1.5% to about 3% of the composition.

9. A method of darkening the skin, said method comprising topically applying to said skin a composition as claimed in any one of claims 1 to 8.

10. Use of a composition as claimed in any one of claims 1 to 8 for darkening the skin.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 12339550 B
- US 5618519 A
- US 5384116 A
- US 5227459 A
- US 5744125 A
- US 5225435 A
- US 5218079 A
- US 5216116 A
- US 7081442 B
- US 7214655 B
- US 6797697 B
- US 7025951 B


### Non-patent literature cited in the description


- **Bobin et al.** *J. Soc. Cosmet. Chem.,* 1984, vol. 35, 265-72
- International Cosmetic Ingredient Dictionary and Handbook. The Cosmetic, Toiletry, and Fragrance Assoc, 2002, 2930-36
- ICI Handbook. 2979-84
- ICI Handbook. 2962-71
- International Cosmetic Ingredient Dictionary and Handbook. The Cosmetic, Toiletry, and Fragrance Assoc, 1997